# EUROPEAN PATENT APPLICATION

(11) **EP 1 898 207 A1**
(43) Date of publication of application: **12.03.2008**
(21) Application number: 06018663.2
(22) Date of filing: 06.09.2006
(51) Int. Cl.: G01N 21/89, G01N 33/34, G06T 7/00, G01B 11/30

(54) **Method and apparatus for measuring intensity of laid lines in a strip-like product**

(71) Applicant: ABB Oy, 00380 Helsinki (FI)
(72) Inventor: Antti, Saarela, 03400 Vihti (FI)

(57) **Abstract**

In a method and apparatus according to the invention, the intensity of a banded pattern in a strip-like product is measured at run-time. The strip-like product progressing at its speed of transport is observed by at least one camera, and at least one digital image (31) comprised of pixels is created for inspection. In the image for inspection, the pixels are arranged in columns, and the columns of pixels are substantially aligned in the direction of transport of the strip-like product. A frequency transform is carried out on at least two columns of pixels within the image for inspection, and a power spectrum (32) is calculated on the basis of the frequency transform. At least one peak location (34) is searched for in the power spectrum, the intensity value of the peak location is calculated (35), and the intensity values of the columns of pixels are used to calculate the average intensity value (36) of the banded pattern.

## Description

### Scope of the invention

The object of the invention is a method according to the preamble of claim 1 and an apparatus according to the preamble of claim 8 for measuring the intensity of a banded pattern in a strip-like product at run-time.

### Prior art

Bands are created in strip-like products in a continuous process such as paper or steel manufacturing. Bands can be created by adding material on top of the basic product or by pressing the basic product.

During the manufacture of cigarette paper, a roll is used to impress a dense banded pattern known as the vergé pattern (papier vergé) onto the paper web. The banded pattern is usually created using a watermark roll in which a metal mould corresponding to the banded pattern impresses the bands on wet paper in the paper machine. The banded pattern is impressed crosswise in relation to the machine direction.

The vergé pattern has a solely visual role. Therefore the banded pattern must be even and its intensity - that is, the strength of the impression of the banded pattern on the paper - must be within the limits set by the customer. The intensity of the banded pattern is usually measured by its opacity, which is a measure of the non-transparency of the paper; non-transparent paper has high opacity. The "high opacity" requirement set by one end customer can typically differ from the "low opacity" requirement set by another end customer by several orders of magnitude.

Common defects in the vergé pattern on cigarette paper include uneven intensity of the banded pattern in the cross machine direction or a weak or completely missing banded pattern. Uneven intensity in the cross-machine direction can be affected by adjusting the compression force of the cylinder that impresses the banded pattern; a defective banded pattern may be caused by various types of roll faults such as dirt or damage to the roll, and the banded pattern will be completely missing if the watermark roll is not lowered onto the paper web at all, for example. In order to make it possible to affect any faults and deficiencies in the banded pattern on the paper roll and improve its quality as quickly as possible, the intensity of the banded pattern must be measured at run-time.

Automatic pattern recognition of strip-like products is often based on CCD line scan cameras that take pictures of light reflected from the surface of the product at different angles. The web itself is moving, so it is sufficient to use the camera to scan consecutive points of the web at high speed. The image signal from the CCD line scan camera is converted to a digital format and digitally processed in an image-processing unit. The end product of the process is a set of electronic images representing selected parts of the strip-like product.

The publication US 6,198,537 B1 presents a method, system and apparatus for verifying the characteristics of moving banded cigarette paper. The method examines the distance between the bands, the width of the bands and the contrast of the banded areas.

The publication US 2005/0115575 A1 presents an apparatus, system and method for the examination of banded cigarette paper. The apparatus measures individual bands on passing cigarette paper at a single point and sends a signal once it has measured a band.

### Description of invention

The purpose of the present invention is to create a method and an apparatus for measuring the intensity of a banded pattern in a strip-like product at run-time. In order to achieve this, the invention is characterised by the features specified in the characteristics sections of claims 1 and 8. Some other preferred embodiments of the invention have the characteristics specified in the dependent claims.

In a method according to the invention, the intensity of a banded pattern in a strip-like product is measured at run-time. In the method the strip-like product is driven at its speed of transport and observed by at least one camera, and at least one digital image comprised of pixels is created for inspection. In the image for inspection, the pixels are arranged in columns. The columns of pixels are substantially aligned in the direction of transport of the strip-like product. A frequency transform is carried out on at least two columns of pixels within the image for inspection, and a power spectrum is calculated on the basis of the frequency transform. At least one peak location is searched for in the power spectrum, and the intensity value of the peak is calculated. The intensity values of peaks calculated for the pixel columns are used to calculate the average intensity value of the banded pattern.

An apparatus according to the invention for measuring the intensity of a banded pattern in a strip-like product includes at least one camera for observing a strip-like product and means for creating at least one digital image for inspection consisting of pixels arranged in columns. The apparatus includes means for carrying out a frequency transform on at least two columns of pixels within the image for inspection, as well as means for calculating a power spectrum from the frequency transform. Furthermore, the apparatus includes means for finding at least one peak location in the power spectrum, means for calculating the peak intensity value at the peak location, and means for calculating the average intensity value of the banded pattern using the peak intensity values calculated for the pixel columns.

The intensity of the banded pattern in the strip-like product is measured at run-time over the entire width of the web. It is advantageous for the manufacturer of the strip-like product if the intensity of the banded pattern and changes in the intensity are known as comprehensively as possible, both in the direction of progress and the crosswise direction of the strip-like product. It is also advantageous if the run-time measurement results can be presented with a precision and scale that corresponds to the precision and scale of any laboratory measurements.

The run-time measurement of the intensity of a banded pattern impressed on a strip-like product that is crosswise to the machine direction is carried out using optical methods. The method and apparatus measure the instantaneous intensity of the cross-banded pattern on the basis of the image for inspection. If desired, the measurement results can be converted to the desired scale, taking into account any optical and process-based variations distorting the measurement results. The produced measurement value can be presented graphically and numerically. A graphical representation allows the band intensity of all strip-like products manufactured during a production shift to be observed as a time-based trend or a cumulative cross-profile, for example. On the other hand, a cross-profile created of instantaneous measurement values enables quick reaction to process disturbances or process control disturbances affecting the band intensity.

The method and apparatus are particularly applicable to imaging the intensity of a continuous crosswise band impressed on cigarette paper, such as a verge pattern. The vergé pattern is created on the paper using a watermark roll at the wet end of the paper machine. On vergé-patterned paper, also known as water-lined paper, the banded pattern contains dense bands parallel to each other in the cross direction of the paper, and the bands are at even intervals in the machine direction. The more transparent parts of the banded pattern, the bands, are impressed, and the band density is typically approximately 9-11 lines per centimetre in the machine direction. The method does not observe an individual missing line but the average intensity of the lines in a fairly large imaged sample. The size of the imaged sample can typically equal one circumference of the watermark roll in the longitudinal direction and one imaging area of the camera in the lateral direction. According to a preferred embodiment, run-time measurement of the intensity of the vergé pattern is carried out after the dryer section of a paper machine manufacturing water-lined paper, before reeling.

The method determines a long uniform image from the image signal for processing. The long image area enables a frequency transform of the sample containing a high number of lines. This will substantially improve the signal-to-noise ratio of the measurement.

The image signal comprises lines of pixels arranged by time, created by the exposure of a moving strip-like product. The camera sensor comprises several light-sensitive elements, and the neighbourhood relations of the data pixels produced by the sensors correspond to the neighbourhood relations of the sensor elements. When the image is created, the lines of pixels are arranged in the same fashion as they were at the moment of imaging. The image can be created one line at a time or from consecutive images. The image signal is digital - that is, an image expressed numerically.

In a preferred embodiment, the strip-like product such as cigarette paper is illuminated at the observation zone. Illumination improves the signal-to-noise ratio. When observing bands impressed on cigarette paper, it is preferable to illuminate the paper web from the side opposite to the cameras at a substantially perpendicular angle. Such an imaging arrangement is generally known as transillumination, and it is advantageous for the observation of paper opacity and changes in opacity.

Measurement of the intensity of the banded pattern is based on frequency analysis in the machine direction. The image used as the basis for measurement is transformed to the frequency plane using Discrete Fourier Transform (DFT), for example. Other generally known frequency transforms allowing the image to be transformed from a spatial representation to the frequency plane, such as Discrete Cosine Transform (DCT), are also suitable. The frequency transform is carried out by sections in the machine direction for each of the camera's pixel elements at a time - in other words, by columns of pixels over the entire image. Any measurement fluctuation in the location of one or more frequency peaks or the lack of a peak is eliminated by separately delimiting each peak corresponding to a pixel column. The frequency peak corresponds to the frequency of the imaged banded pattern and may be completely missing due to partial contamination of the camera lens, for example. Column-specific processing allows such sections to be ignored in the determination of average intensity. The peak location may also be slightly shifted - that is, the observed frequency of the banded pattern is not constant over the entire width of the image. This can be caused by high-frequency vibration of the measurement frame, for example. Column-specific processing allows each peak corresponding to the banded pattern to be determined separately, eliminating the effects of various disturbances.

The power spectrum of the sample is determined in the frequency plane. The power spectrum can be obtained by calculating the square of the absolute value of the Fourier transform, for example. The power spectrum can be represented visually, for example as a graph in which the horizontal axis represents the equally spaced frequency bands in increasing order, and the vertical axis represents the power in each frequency band measured from the sample. A frequency band refers to a continuous range of frequencies limited at the bottom and top. The bands are in a continuous evenly increasing order, so that each observable frequency is reflected in exactly one frequency band. The section of the graph corresponding to each frequency band represents the power of that frequency band in the image. The more intense waves corresponding to the frequency band are existing in the image, the higher the value of the frequency band in the power spectrum. The narrowest useful width of the frequency bands in the power spectrum is naturally determined by the number of pixel rows in the image being examined. The power spectrum can be divided into equally spaced frequency bands so that the number of bands is one-half of the number of pixel rows in the image - that is, the height of the image. This preserves all of the information obtained from the frequency transform. Analysis of the power spectrum will reveal any banded pattern in the sample and its intensity.

The highest peak in the power spectrum is assumed to correspond to a regular banded pattern in the sample. In this context, a peak refers to a power spectrum value that is substantially higher than its neighbourhood, the neighbourhood being considered as a few adjacent bands in the power spectrum in the directions of both increasing and decreasing frequency. The location of the highest peak in the power spectrum is determined by applying low-pass filtering to the power spectrum using a median filter, for example, and subsequently subtracting the low-pass filtered spectrum from the original, creating a difference signal. It is preferable to carry out the low-pass filtering using a window width that at least exceeds the assumed maximum width of a peak. This way the low-pass filtered signal will represent the variation of background in the image and measurement noise. In the case of cigarette paper, most of the background noise is caused by optical variation of the formation of the paper. The subtraction of the low-pass filtered spectrum from the power spectrum eliminates the effect of background noise, and the desired highest peak and corresponding frequency band can be found by finding the highest value of the difference signal. Because the crosswise band in vergé paper resembles a rectangular wave rather than a sine wave, its response in the frequency plane is spread over a section of a certain width. The highest peak of the filtered power spectrum is assumed to be close to the middle bands within the section. Determination of the peak location within the power spectrum enables the next phase, the determination of power within the band section corresponding to the banded pattern.

The power spectrum peak location can also be used for verifying the validity of the results. When the sample rate of the imaging camera, the speed of the paper machine and the density of the banded pattern on the paper are known, the assumed location of the peak in the frequency plane can be calculated in advance. The density of the banded pattern and the running speed of the paper machine can be used to calculate the frequency of occurrence of the banded pattern. This indicates the number of bands per second passing the scan line of the camera. The interval between bands within the image in pixels can be calculated by dividing the sample rate of the camera - that is, the number of pixel rows per second produced by the camera - with the frequency of the banded image. This allows advance determination of the assumed location of the peak produced by the banded pattern in the power spectrum. If no observable peak can be found in the frequency band in question, it can be stated that there is no banded pattern in the sampled area or that imaging has failed (for example, at the time of imaging, the paper web was interrupted, the camera lens was contaminated, the camera was wrongly focused or the like). However, if the highest peak is found within another frequency band, the information regarding the speed of the paper machine can be incorrect or inaccurate.

According to an embodiment, a frequency band zone is generated from the frequency of occurrence of the banded pattern, with the lowest frequency of the zone being more than 90% of the anticipated frequency of occurrence, and the highest frequency of the zone being less than 110% of the anticipated frequency of occurrence. An average intensity value of the banded pattern is calculated within this frequency band zone using the intensity values of peaks calculated from the pixel columns.

When the location of the frequency plane peak caused by the banded pattern is known, the intensity of the banded pattern is determined by summing the values of the difference signal within the frequency band corresponding to the peak location. The width of the frequency band corresponding to the summation - in other words, the peak and its neighbourhood - is determined as a continuous frequency band within which the power in the frequency band does not fall below a specified percentage of the highest peak power for a higher number of consecutive frequency bands than has been determined in advance. The minimum power for a frequency band - in other words, the limits of summation in the directions of increasing and decreasing frequency - within the neighbourhood of the peak can be set as ten per cent of the peak power value, for example, and the maximum number of consecutive frequency bands lower than this can be set as three. The limits to be set depend on the noise level of the measurement and the background paper, as well as the frequency band resolution being used. On the other hand, the peak intensity in the frequency plane decreases steeply in the neighbourhood of the highest peak, so it is not necessary to accurately determine the bandwidth with regard to the precision of the band intensity measurement. The value representing the intensity of the banded pattern obtained through summation is the desired measurement result for the pixel column in question.

Once all pixel columns have been calculated, the measurement result representing the sample is calculated from the power values produced by each pixel column. This is carried out by calculating a statistical quantity representing average intensity, such as median or average, over the pixel-specific values. Higher-order statistical quantities, such as variance, can be derived from the results and used to observe the precision of the measurement configuration and equipment. When the long-term average and variance specific to each pixel column produced by the measuring equipment are known, the results specific to each pixel column can be used for the observation of the uniform quality of the banded pattern in the cross direction with high crosswise precision.

The achieved measurement result is affected by various types of interference, some of which can be compensated for in the final results by using stored measurement values. Optical non-idealities causing the greatest variations include imperfect focusing of cameras varying from one camera to another, the alignment of the line scan camera's sensor element in relation to the crosswise direction of the web, the location of the camera's viewing area in the machine direction in relation to the stripe of light created by the light beam, uneven intensity of the light pattern created by the light beam in the crosswise direction, as well as the commonly known "cosine to the fourth" phenomenon within the viewing area of a single camera that impairs the lighting level and therefore the contrast between a crosswise band and the background towards the edges of the camera's viewing area. In addition to optical non-idealities, the measurement is also affected by the speed of the machine, potential flutter of the web in the vertical direction, movement of the web's edge area in the crosswise direction, the quality of paper produced and variation of the optical properties of the paper due to natural variation of the production process.

Variation of formation of the paper, noise associated with imaging and the distortions mentioned above cause a lot of variance in individual measurement points within the image. Due to this, it is preferable to divide the camera's viewing area into a number of sections within which the results of the measurement points are averaged. The sections can be partially overlapping or adjacent, and averaging can be carried out by calculating an average or median, for example. The use of sufficiently wide sections brings the noise level of the measurement to an acceptable level. One measurement result per each round of imaging is calculated for each of the sections formed. During the round of imaging, the process goes through all of the cameras, and the entire combined viewing area is imaged.

Imbalances between the sections can be observed in long-term averages. Optical distortions will remain relatively constant in relation to each other as long as the measurement configuration is unchanged. Unchanged optical distortions of measurement results are calibrated by storing the results for each section over a certain period of time and using the averages of the results to determine the calibration coefficients used for normalising the results.

The calibration of distortions caused by the imaging system will also balance out any irregularities in the crosswise profile of the measured object that remain unchanged over the calibration period. In order to be able to reproduce these, the shape of the actual crosswise profile determined from laboratory samples, for example, is taken into account in the calibration of the apparatus. By providing the apparatus with the actual crosswise profile as correction coefficients, the even response of the meter can be shaped to correspond with reality. If the laboratory sample represents a known point in time, the optical distortions of the meter are compensated for close to that time. If the actual crosswise profile cannot be determined for any point in time, the apparatus will detect any changes in the profile, and the measurement values can still be used for monitoring changes in relation to time.

Distortion of measurement results associated with the production process can also be corrected using correction coefficients. Because the method is particularly well suited for measurement based on very long images stored using a line scan camera, the running speed of the machine substantially affects the measurement results. The effect of machine speed can be minimised in a similar way to optical distortions. Increasing running speed will be manifested as low-pass filtering of the banded image in the machine direction, and the peak location in the power spectrum will move towards increasing frequency. The height and shape of the peak will change with running speed, and the changes will be reflected in the measurement results as well. Long-term history or laboratory conditions can be used to determine the impact of speed on the measurement results, and data on the speed distortion can be provided to the meter in the form of tables, for example. The distortion data allows the compensation of the impact of running speed on the results.

The method and apparatus produce measurement results on the intensity of the crosswise band immediately during run-time, so they can be used to improve the quality of the roll currently being processed. For example, if asymmetry is detected in the banded pattern in relation to the crosswise direction of the web, the compression force of the roll impressing the banded watermark can be adjusted at different sections of the roll, for example at the edges and middle of the roll. If the entire banded pattern is found to be missing from the paper, it is possible that the watermark roll has not been lowered onto the paper web, and the situation can be rectified. A defective banded pattern may be caused by various types of roll fault, such as dirt or damage to the roll.

The measurement results also serve as an indicator of the condition of the roll impressing the crosswise band. The watermark roll impressing the crosswise band, also known as the dandy roll, is typically washed at approximately one-week intervals. Maintenance will naturally require production stoppage. The measurement results allow optimisation and appropriate timing of the maintenance interval, resulting in cost savings and the maximisation of production. Gradual weakening of the crosswise banded pattern will be clearly visible in a time-based trend drawn from the measurement results, and can be used for long-term quality control in addition to the planning of maintenance intervals. For this purpose, the method produces substantially more measurement points compared to traditional laboratory analysis that can practically be carried out only on a few batches of samples taken from the end of the roll that span the entire web.

The presented method works and also produces results in a situation where the sample rate of the imaging camera is slower than the frequency of occurrence of crosswise bands going under the camera on the paper web. In this case, the nominal values of the results produced by the method will decline and the resolution capability of intensity differences in the crosswise band will slightly weaken as the frequency of band occurrence increases.

The method works for all line densities of a banded pattern that allow a sufficient number of lines to be stored in the image or image signal for processing at each time. The precision of measurement will increase with an increased number of lines. Two or more densities of banded patterns can also be measured simultaneously if they are sufficiently far away in the frequency plane - that is, at different densities from each other. For example, an application of the invention can be used for measuring the intensity of the verge pattern and the extinguishing stripe added to cigarette paper at certain intervals. The extinguishing stripe differs from the vergé pattern only by its transilluminated intensity and the width and density of the bands.

The method and apparatus for measuring the intensity of a banded pattern in a strip-like product at run-time is applicable to various different manufacturing processes of strip-like products, such as the manufacture of cold- or hot-rolled stainless steel, the manufacture of aluminium or copper band, and the manufacture of paper and board.

### Figures

In the following the invention will be described in more detail with the help of certain embodiments by referring to the enclosed drawings, where
- Figure 1 is a general illustration of the arrangement for measuring the intensity of a banded pattern at run-time;
- Figure 2 illustrates a long image in the measurement of the intensity of a banded pattern;
- Figure 3 is a block diagram of the method for measuring the intensity of a banded pattern in a strip-like product at run-time combined with compensation for interference;
- Figure 4 illustrates the power spectrum;
- Figure 5 illustrates the difference signal.

### Detailed description

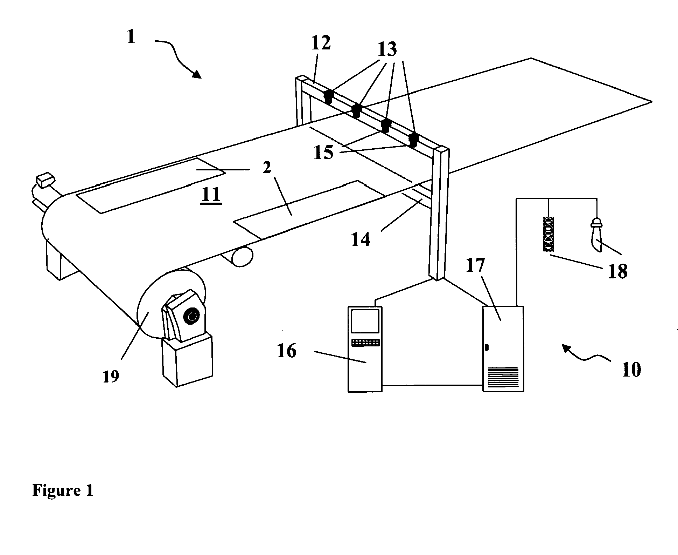

Figure 1 illustrates an arrangement for measuring the intensity of a banded pattern in a strip-like product at run-time, allowing the use of the method and apparatus for measuring the intensity of a banded pattern in a strip-like product at run-time. The apparatus is applicable to various types of continuous and discontinuous production lines. Figure 1 illustrates a measurement arrangement 1 for vergé-patterned cigarette paper, in which the system 10 measures the intensity of the banded pattern on the cigarette paper 11 on the paper machine before reeling 19. To make Figure 1 clearer, the dense vergé pattern is not illustrated in Figure 1.

The moving paper web 11 is inspected by one or more cameras 13 on one side of the paper web 11. The cameras 13 are fitted to a suitable mechanical support, such as a camera beam 12. The paper web 11 is transilluminated from below using a light source 14. The light source can also be located over the paper web 11. Penetrating light is primarily used for transparent materials as illustrated in Figure 1. Reflected light is suitable in particular for other types of materials. In the case of reflected light, the lighting angle can be specular or scattered in relation to the camera viewing angle.

The cameras 13 can be any type of electronic cameras that can be directly or indirectly connected to an image processing unit 15. The functions of the image processing unit 15 can also be integrated with the camera 13, in which case the camera 13 is a more complex and independent image processing unit. The image signal from an analogue camera, such as an analogue CCD line or matrix camera, must first be converted into a digital form. The image data produced by a digital camera is usually better suited for digital processing in the image processing unit 15. The image processing unit 15 receives from the cameras 13 a digital representation of the view imaged by the cameras 13. The representation is in the form of a series of digital numbers. The image processing unit 15 interprets the material as an electronic image, referred to as an image elsewhere in this context, on the basis of information it has about the properties of the camera 13. For example, the image processing unit 15 combines the sequential data series sent by a line scan camera into a matrix that represents an image of the paper web 11. Long images 2 of the paper web are created and used for run-time measurement of the intensity of the banded pattern.

The image processing unit 15 is a separate unit of equipment that is usually programmable. It can be partially or fully integrated in the camera as illustrated in Figure 1. It can also be a personal computer or some other computer of a common type. One computer carries out the processing of image material from one or more cameras. The final product of this stage of processing is a set of electronic images representing selected parts of the web. The images are electronically manipulated to fulfil the requirements of the application at hand.

The images are forwarded to the next stage of processing, image analysis. This stage can be carried out using a separate computer which can be a part of the workstation 16 within the system 10 and which is usually shared between all of the cameras 13.

The workstation 16 includes the user interface for the system 10. It is used for the entry of different control parameters and the selection of desired views and reports that may indicate the state of the system and the quality of the inspected products, for example. The visual inspection system 10 naturally requires separate means for supplying power to the system and equipment for connecting with external systems, such as the actual process. These means, which are obvious to a person skilled in the art, can be located in an electrical cabinet 17. In addition to the workstation 16, external devices 18 can be used that provide warnings to the operator.

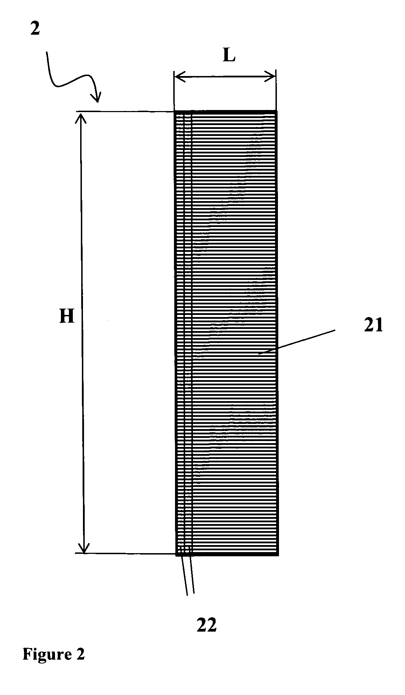

Figure 2 illustrates a long image 2 used for run-time measurement of the intensity of a banded pattern. Figure 2 is taken of the cigarette paper web 11 of Figure 1 before reeling 19 using the CCD line scan camera 13. There are four cameras in Figure 1, and one-half of a camera is examined at a time. The typical width of a cigarette paper web is 3-5 m and the typical running speed of a modem cigarette paper machine is 400-450 m/min. A preferable imaging rate for the camera is 16 kHz per imaged line. The width L of the image being examined is 0.15 m and its length H in the machine direction is 4 m. While the crosswise band on the cigarette paper being examined includes 11 bands per centimetre in the machine direction, there are 4400 bands 21 in Figure 2. The long image being examined includes 512 parallel columns of pixels 22; Figure 2 only illustrates two of these to make the figure clearer. The columns of pixels are substantially aligned in the direction of transport of the strip-like product.

The numerical values presented in connection with Figure 2 are examples and vary by application and measurement configuration.

The measurement method is also suitable for the measurement of sparsely banded patterns if a sufficient number of lines can be stored in the picture at each time. For example, if there are lines at one-metre intervals in the banded pattern, the method is suitable for the measurement of their intensity if the length of the image is approximately 10 metres.

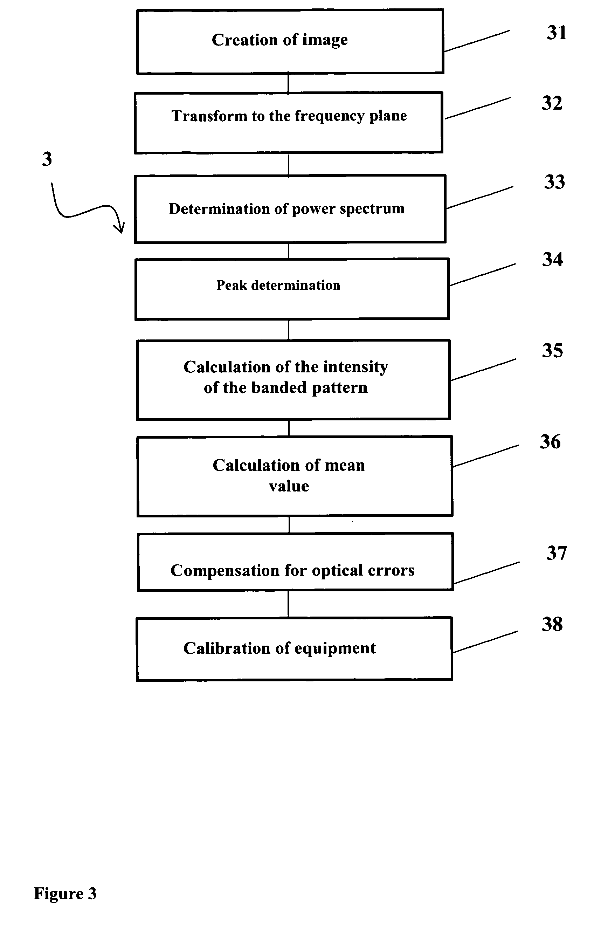

Figure 3 illustrates a block diagram of the method for measuring the intensity of a banded pattern in a strip-like product at run-time combined with the compensation of interference. At the first phase 31 of the method a digital long image of the object being examined is created. Figure 2 shows an example of a long image.

At the next phase 32 the image created of the object is transformed to the frequency plane using Discrete Fourier Transform (DFT), for example. Other generally known frequency transforms allowing the image to be transformed from a spatial representation to the frequency plane, such as Discrete Cosine Transform (DCT), are also suitable. The frequency transform is carried out by sections for each of the pixel elements at a time in the machine direction - in other words, by columns of pixels. Any measurement fluctuation in the location of one or more frequency peaks in the frequency plane is eliminated by separately delimiting each frequency peak.

At phase 33 the power spectrum of the sample - that is, one pixel column - is determined in the frequency plane. The power spectrum illustrates the distribution of signal power over different frequencies. Analysis of the power spectrum will reveal any banded pattern in the sample and its intensity.

At phase 34 the location of the highest peak in the power spectrum is determined by low-pass filtering of the power spectrum using a median filter, for example, and subsequently subtracting the low-pass filtered spectrum from the original. It is preferable to carry out the low-pass filtering using a window width that at least exceeds the assumed maximum width of a peak. This way the low-pass filtered signal will represent the variation of background in the image and measurement noise. In the case of cigarette paper, most of the background noise is caused by optical variation of formation of the paper. The subtraction of the low-pass filtered spectrum from the power spectrum eliminates the effect of background noise, and the desired highest peak and corresponding frequency band can be found by finding the highest value of the difference signal. Because the crosswise band resembles a rectangular wave rather than a sine wave, its response in the frequency plane is spread over a section of a certain width. The highest peak of the filtered power spectrum is assumed to be close to the middle bands within the section. Determination of the peak location within the power spectrum enables the next phase, the determination of power within the band section corresponding to the banded pattern.

When the location of the frequency plane peak caused by the banded pattern is known, the intensity of the banded pattern is determined by summing the values of the power spectrum or difference signal within the frequency band corresponding to the peak location; phase 35. The width of the frequency band corresponding to the summation - in other words, the peak and its neighbourhood - is determined as a continuous frequency band within which the power in the frequency band does not fall below a specified percentage of the highest peak power for a higher number of consecutive frequency bands than has been determined in advance. The minimum power of a frequency band within the neighbourhood of the peak can be set as ten per cent of the peak power value, for example, and the maximum number of consecutive frequency bands lower than this can be set as three. The limits to be set depend on the noise level of the measurement and the background, as well as the frequency resolution being used. On the other hand, the peak intensity in the frequency plane decreases steeply in the neighbourhood of the highest peak, so it is not necessary to accurately determine the bandwidth with regard to the precision of the band intensity measurement. The value representing the intensity of the banded pattern obtained through summation is the desired measurement result for the pixel column in question.

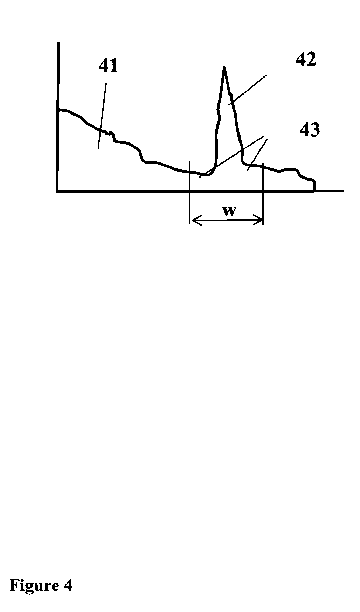

In Figure 4, the power spectrum 41 is represented as a histogram in which the horizontal axis represents the equally spaced frequency bands in ascending order and the vertical axis represents the power in each frequency band measured from the sample. The width w of the frequency band corresponding to summation at phase 35, in other words the peak 42 and the neighbourhood 43 of the peak, is defined to cover the area corresponding to the power of the entire banded pattern.

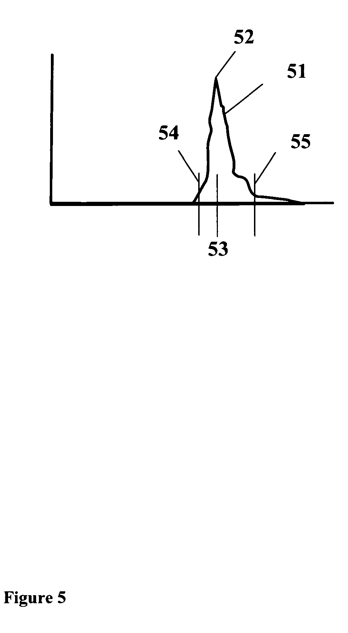

Figure 5 illustrates the difference signal 51. The peak location in the difference signal is determined by choosing the highest value 52 of the difference signal; the corresponding frequency band 53 is the peak location. The intensity value of the peak 52 is determined by using the intensity value of the frequency band 53 corresponding to the location of the peak 52 to calculate at least one limit 55 in the direction of increasing frequency, and at least one limit 54 in the direction of decreasing frequency. The limits 54, 55 are substantially lower than the intensity value of the frequency band 53 corresponding to the location of the peak 52, for example as low as 8-12%. Intensity values are calculated for consecutive frequency bands from the frequency band 53 corresponding to the location of the peak 52 towards increasing and decreasing frequency. When the calculated intensity of the frequency band in the direction of increasing frequency is lower than the limit 55 for the increasing frequency, the frequency band intensities calculated in the direction of increasing frequency are summed together. When the calculated intensity of the frequency band in the direction of decreasing frequency is lower than the limit 54 for the decreasing frequency, the frequency band intensities calculated in the direction of decreasing frequency are summed together. Finally, the sums calculated towards increasing and decreasing frequency and the intensity value of the frequency band 53 corresponding to the location of the peak 52 are summed together.

At phase 36 all of the pixel columns (512 in Figure 2) have been calculated, and the results are used to calculate an average value with the selected frequency band width. The average value can be a median or an average, for example.

The achieved measurement result is affected by various types of interference, some of which can be compensated for in the final results, if desired, by using stored measurement values. At phase 37 deviations due to optical distortions are compensated for by storing the results for each section over a certain period of time and using the averages of the results to determine the calibration coefficients used for calibrating the results.

The calibration of distortions caused by the imaging system will also eliminate any irregularities in the crosswise profile of the measured object. In order to detect and represent these, the apparatus is calibrated using an actual crosswise profile obtained from laboratory samples, for example; phase 38. By providing the apparatus with the actual crosswise profile as correction coefficients, the even response of the apparatus can be shaped to correspond with reality. If the laboratory sample represents a known point in time, the optical distortions of the meter are compensated for close to that time. If the actual crosswise profile cannot be determined for any point in time, the equipment will detect any changes in the profile, and the measurement results can still be used for monitoring changes in relation to time.

The invention has been described above with the help of certain embodiments. However, the description should not be considered as limiting the scope of patent protection; the embodiments of the invention may vary within the scope of the following claims.

## Claims

1. A method for measuring the intensity of a banded pattern in a strip-like product at run-time, in which method a strip-like product (11) is driven at its speed of transport and observed by at least one camera (13), and in which at least one digital image (2) consisting of pixels is created for inspection, the pixels in the image (2) for inspection being arranged in columns (22), the columns of pixels (22) being substantially aligned in the direction of transport of the strip-like product, **characterised in that** a frequency transform is carried out on at least two columns of pixels (22) within the image for inspection, the frequency transform is used to calculate a power spectrum (41), at least one peak location (42) is searched for in the power spectrum (41), the intensity value of the peak (42) is calculated, and the intensity values of the peaks (42) calculated from the columns of pixels (22) are used to calculate the average intensity value of the banded pattern.

2. A method according to Claim 1, **characterised in that** the calculated power spectrum (41) is low-pass filtered and the low-pass filtered power spectrum is subtracted from the power spectrum (41), creating a difference signal (51), the highest value (52) is selected from the difference signal, and the frequency band (53) of the selected value is the peak location.

3. A method according to any of the Claims from 1 to 2, **characterised in that** the intensity value of the peak (42, 52) is determined by calculating at least one limit (55) of the intensity value of the frequency band (53) corresponding to the location of the peak (42, 52) in the direction of increasing frequency and at least one limit (54) in the direction of decreasing frequency, the limits being lower than the intensity value of the frequency band (53) corresponding to the location of the peak (42, 52), and by calculating the intensity values of consecutive frequency bands from the frequency band (53) corresponding to the location of the peak (42, 52) in the directions of increasing and decreasing frequency; when the calculated intensity of the frequency band in the direction of increasing frequency is lower than the limit (55) for the increasing frequency, the frequency band intensities calculated in the direction of increasing frequency are summed together, creating an increasing sum, and when the calculated intensity of the frequency band in the direction of decreasing frequency is lower than the limit (54) for the decreasing frequency, the frequency band intensities calculated in the direction of decreasing frequency are summed together, creating a decreasing sum, and the increasing sum, decreasing sum and the intensity value of the frequency band corresponding to the location of the peak (42, 52) are then summed together.

4. A method according to Claims 1 to 3, **characterised in that** the strip-like product (11) is illuminated (14) at the observation zone.

5. A method according to any of the Claims from 1 to 4, **characterised in that** the transport speed of the strip-like product and the intervals between the bands in the banded pattern are used to calculate the frequency of occurrence of the bands, the frequency of occurrence is used to generate a frequency band zone with the lowest frequency of the frequency band zone being more than 90% of the frequency of occurrence, and the highest frequency of the frequency band zone being less than 110% of the frequency of occurrence, and an average intensity value within the frequency band zone is calculated from the intensities of the peaks (42, 52) calculated from the columns of pixels (22).

6. A method according to any of the Claims from 1 to 5, **characterised in that** the strip-like product is water-lined paper (11) manufactured on a paper machine and that the water-lined paper is observed by a camera (13) when it moves between the dryer section of the paper machine and reeling (19).

7. A method according to any of the Claims from 1 to 6, **characterised in that** the peak (42, 52) intensity measurement results are multiplied by correction coefficients to correct optical distortions and distortions in the measurement results associated with imaging and the production process that are unchanged in the long term.

8. An apparatus for measuring the intensity of a banded pattern in a strip-like product, the apparatus including at least one camera (13) for observing the strip-like product and means for creating at least one digital image (2) for inspection consisting of pixels arranged in columns (22), **characterised in that** the apparatus includes means for carrying out a frequency transform on at least two columns of pixels (22) within the image for inspection, means for calculating a power spectrum (41) from the frequency transform, means for finding at least one peak location (42) in the power spectrum, means for calculating an intensity value at the peak location (42), and means for calculating an average intensity value of the banded pattern from the intensity values of the peaks calculated from the columns of pixels (22).

9. An apparatus according to Claim 8, **characterised in that** the apparatus comprises means (14) for illuminating (11) a moving strip-like product at the observation zone.
